# EUROPÄISCHE PATENTSCHRIFT

(11) **EP 0 687 505 B1**
(45) Veröffentlichungstag und Bekanntmachung des Hinweises auf die Patenterteilung: **03.11.1999**
(21) Anmeldenummer: 95108882.2
(22) Anmeldetag: 09.06.1995
(51) Int. Cl.: B03C 1/28, G01N 33/543

(54) **Verfahren zur magnetischen Abtrennung von Flüssigkeitskomponenten**
Magnetic separation method for components of a liquid
Méthode de séparation magnétique pour des composants d'un liquide

(30) Priorität: 16.06.1994 DE 4421058
(43) Veröffentlichungstag der Anmeldung: 20.12.1995
(73) Patentinhaber: Roche Diagnostics GmbH, 68298 Mannheim (DE)
(72) Erfinder: Bienhaus, Gerhard, Dr., D-82407 Wielenbach (DE); Stolz, Burkhard, D-82386 Huglfing (DE); Schubert, Ulrich, D-82319 Starnberg (DE)

(56) Entgegenhaltungen:
- EP-A- 0 125 995
- EP-A- 0 140 787
- WO-A-87/05536
- US-A- 5 043 063

## Beschreibung

Gegenstand der Erfindung ist ein Verfahren zur Abtrennung einer Komponente einer Flüssigkeit von anderen Komponenten der Flüssigkeit sowie eine dafür geeignete Vorrichtung.

Bei der Analyse von Komponenten einer Flüssigkeit, insbesondere von Inhaltsstoffen von Körperflüssigkeiten, stellt sich oft das Problem, daß die Flüssigkeit nur kleine Mengen der Komponente enthält und außerdem noch eine Reihe ganz ähnlicher Komponenten vorhanden sind. Aus diesem Grund wird schon seit längerer Zeit der eigentlichen Detektion der Komponente ein Schritt zur Anreicherung beziehungsweise Reinigung der nachzuweisenden Komponente vorgeschaltet. Ein gerne beschrittener Weg ist die möglichst selektive Immobilisierung der Komponente an einer festen Phase und anschließende Abtrennung der Flüssigkeit mit den ähnlichen Komponenten. Die immobilisierten Komponenten werden dann entweder an der festen Phase direkt oder nach Überführung in eine weitere Flüssigkeit nachgewiesen. Als feste Phase können saugfähige Vliese, Behälterwandungen oder auch teilchenförmige Phasen verwendet werden.

In jüngerer Zeit hat man magnetische oder magnetisierbare Teilchen als feste Phasen zu schätzen begonnen, da diese wegen ihrer Suspendierbarkeit eine ausreichend schnelle Immobilisierung der Komponenten an ihre Oberfläche erlauben, andererseits aber auch durch Anlegen eines Magnetfeldes schnell von sie umgebenden Flüssigkeiten zu separieren sind. In der EP-A-0 589 636 ist ein Beispiel für ein Verfahren zur magnetischen Separierung von Magnetteilchen beschrieben. Bei diesem Verfahren wird das Gefäß, welches die Teilchen enthält, in eine Vorrichtung eingebracht, die in einem unter dem Gefäß liegenden teil einen oder mehrere Stabmagneten aufweist, so daß die magnetischen Teilchen im unteren Teil der Innenwand des Gefäßes gesammelt werden können.

In WO 92/04961 ist ein Verfahren beschrieben, bei dem die von außen wirkenden Magnetkräfte durch Eintauchen eines magnetisierbaren Drahtes verstärkt werden. Die magnetischen Partikel werden an den Drähten festgehalten. Anschließend können die Drähte aus den Gefäßen entfernt, gewaschen und die an ihnen immobilisierte Menge der Komponente bestimmt werden.

In EP-A-0 339 980 ist ebenfalls ein Verfahren zur Separierung magnetischer Partikel von umgebenen Flüssigkeiten beschrieben, bei dem durch außen an eine Pipette angebrachte Magnete magnetische Teilchen an einem in der Pipette befindlichen Draht immobilisiert werden. Nach Waschen der immobilisierten Teilchen werden diese durch Vibration der Drähte resuspendiert.

In EP-A-0 265 244 ist ein Verfahren zum Abfangen von nachzuweisenden Substanzen an Magnetpartikeln und die Entfernung der Magnetpartikel von der die nachzuweisenden Substanzen enthaltenden Flüssigkeit beschrieben.

Für die Zellseparation werden auch Vorrichtungen in Filterform mit integrierter Stahlwolle zur Magnetverstärkung (DE-) oder scharfkantigen Ferriten (DE-A-42 44 369) verwendet.

Für eine Automatisierung sind diese Verfahren wenig geeignet, da entweder das Röhrchen transportiert werden muß (WO 93/25912) oder die Magnete an die Reaktionsgefäße herangebracht werden müssen. In WO 92/16844 ist ein Automatisierungsansatz beschrieben, wobei eine erhebliche Kontaminationsgefahr besteht und die Resuspendierung nicht ausreichend ist. Auch hier sind die Magnete außerhalb der Reaktionsgefäße angeordnet.

Ein Nachteil der bisher beschriebenen Verfahren ist, daß sie eine komplizierte und oft wenig effektive Resuspendierung erfordern. Einmal in eine Probenflüssigkeit eingetauchte Drähte sind nicht ohne weiteres wiederverwendbar.

In der WO 87/05536 ist eine Vorrichtung zum Abtrennen und Resuspendieren magnetischer Partikel offenbart, die analog zu der vorliegenden Erfindung eine Schutzhülle aufweist, die in eine Suspension eingetaucht wird, und auf der durch Einführen eines Magneten in die Schutzhülle die in der Suspension enthaltenden magnetischen Partikel abgeschieden werden. Zur weiteren Bearbeitung der abgeschiedenen Partikel werden diese zusammen mit der Abscheidevorrichtung in ein anderes Testgefäß überführt (siehe Seite 3, Zeilen 27 bis 32).

Aufgabe der vorliegenden Erfindung war es daher, ein Verfahren zur Verfügung zu stellen, bei dem die Abtrennung von Magnetpartikeln effektiv ist und bei dem die zur Immobilisierung verwendeten Vorrichtungsteile vor Kontamination geschützt sind.

Gegenstand der Erfindung ist daher ein Verfahren zur Abtrennung einer Komponente einer Flüssigkeit von anderen Komponenten durch Immobilisierung der Komponente an magnetischen suspendierten Teilchen in einem nach unten hinzu öffnenden Gefäß, Eintauchen einer magnetischen Vorrichtung in das Gefäß, wobei die Vorrichtung gegenüber der Flüssigkeit durch eine Schutzhülse aus nicht magnetischem Material abgetrennt ist und Entfernen nicht immobilisierter Komponenten durch Absaugen oder Ablassen der Flüssigkeit aus dem Gefäß. Ebenfalls Gegenstand der Erfindung sind ein Verfahren zum Nachweis der Komponente und eine Vorrichtung zur Durchführung dieses Verfahrens.

Unter Flüssigkeiten im Sinne der Erfindung sind insbesondere Flüssigkeiten zu verstehen, wie sie in der klinischen Analytik anfallen. Dazu gehören insbesondere Körperflüssigkeiten wie Blut, Serum, Plasma, Lymphflüssigkeit, Stuhl, Sputum oder Urin sowie davon abgeleitete Flüssigkeiten. Abgeleitete Flüssigkeiten sind solche, die durch Zugabe von Reagenzien oder durch Entnahme einzelner Komponenten hergestellt wurden. Komponenten dieser Flüssigkeiten sind insbesondere chemische Substanzen mit einer analytischen Bedeutung, wie zum Beispiel immunologisch aktive Substanzen, zum Beispiel Antikörper, Antigene, Haptene, aber auch Nukleinsäuren, zum Beispiel aus Zellen des betreffenden Körpers, jedoch auch Organismen, die diesen Körper befallen haben, zum Beispiel Viren oder Bakterien, sowie Zellen, zum Beispiel des Abwehrsystems des Körpers, wie Lymphozyten. All diese Komponenten liegen in den Flüssigkeiten im Gemisch mit anderen Komponenten vor, von denen sie sich oft nur geringfügig unterscheiden.

Die erfindungsgemäße Vorrichtung ist bei der Probenvorbereitung bei Nukleinsäuretests besonders gut einsetzbar.

Im erfindungsgemäßen Verfahren wird auf im Prinzip bekannte Weise eine Wechselwirkung der abzutrennenden Komponente mit einem Reaktionspartner zur Bindung an ein magnetisches Teilchen ausgenutzt. Solche Wechselwirkungen hängen von der Komponente ab. Besonders geeignet sind die immunologischen Wechselwirkungen wie zwischen Antikörpern und Antigenen sowie die Hybridisierungsneigung von Nukleinsäuren mit im wesentlichen dazu komplementären Nukleinsäuren. Automatisierte Durchführungen von Reaktionen mit Magnetpartikeln sind in unterschiedlichsten Ausführungen bekannt, zum Beispiel aus US-A 4,233,169, WO 83/03920, US-A 3,970,518 oder US-A 4,672,040. Die hieraus bekannten Bedingungen zur Immobilisierung einer Komponente aus einer Flüssigkeit sind auf die vorliegende Erfindung übertragbar.

Unter einem Gefäß wird ein Behälter verstanden, welcher die Flüssigkeit sowie die darin enthaltenen Komponenten enthält. Erfindungsgemäß handelt es sich um ein nach unten hin zu öffnendes Gefäß handeln. Diese Gefäße bestehen aus einem nicht magnetischen und nicht magnetisierbaren Material, zum Beispiel Glas oder Kunststoff, bevorzugt Polystyrol, Polyethylen, Polypropylen, Polycarbonat oder Polyurethan. Bevorzugt faßt dieses Gefäß einen Inhalt von zwischen 50 µl und 10 ml, besonders bevorzugt zwischen 200 µl und 2 ml. Die Gefäße haben bevorzugt eine im wesentlichen zylindrische Form, wobei die Länge größer ist als der Durchmesser.

Unter magnetischen Teilchen sollen insbesondere Mikropartikel verstanden werden, die durch ein magnetisches Feld angezogen werden. Sie können demnach selbst eine Magnetisierung besitzen. Bevorzugt sind jedoch solche Materialien, die eine möglichst kleine Remanenz aufweisen. Das Material der Teilchen kann ein zusammengesetztes Material sein, beispielsweise eine Matrix, die magnetisch anziehbare Teilchen enthält. Bei dem magnetisch anziehbaren Material kann es sich beispielsweise um Eisen, Eisenoxide, Nickel-, Kobalt- oder Chromoxid handeln. Die Matrix kann aus einer Vielzahl von Materialien, zum Beispiel organischen oder anorganischen Polymeren, bestehen. Die magnetischen Teilchen sind mit einem Bindepartner für die abzutrennende Komponente beschichtet. Sowohl Herstellung von magnetischen Teilchen als auch deren Beschichtung mit immunologisch aktiven Verbindungen oder kovalente Verknüpfung mit Nukleinsäuren ist aus dem Stand der Technik bekannt, zum Beispiel aus der US-A 4,297,337 oder DE-A-30 14 036. Solche Partikel sind auch im Handel erhältlich, zum Beispiel von Dynal oder Rhone-Poulenc. Die letztgenannten Teilchen sind für das erfindungsgemäße Verfahren von Vorteil, da sie relativ stabile Suspensionen bilden.

Im ersten Schritt des erfindungsgemäßen Verfahrens wird die die Komponente enthaltende Flüssigkeit mit den magnetischen Teilchen in Kontakt gebracht. Die Teilchen liegen während der Inkubation bevorzugt im suspendierten Zustand vor. Durch die Wechselwirkungen der Oberfläche der Teilchen mit der abzutrennenden Komponente findet die Immobilisierung der Komponente an den Teilchen statt. Dieser Reaktionsschritt findet in einem oben genannten Gefäß statt.

Nachdem eine ausreichende Menge der abzutrennenden Komponente an den Teilchen immobilisiert ist, wird erfindungsgemäß eine magnetische Vorrichtung in das Gefäß eingetaucht. Dabei soll mindestens ein Teil der Vorrichtung bis unter die Oberfläche der Flüssigkeit hineinragen. Erfindungsgemäß ist die Vorrichtung gegenüber der Flüssigkeit durch eine Schutzhülse aus nicht magnetischem Material abgetrennt. Diese Schutzhülse kann beispielsweise aus Kunststoffen bestehen. Das Eintauchen der Vorrichtung und der Schutzhülse kann prinzipiell auf zwei Weisen geschehen. In einer ersten Ausführungsform wird zunächst die Schutzhülse in die Flüssigkeit im Gefäß eingetaucht, so daß ein Teil des durch die Schutzhülse gebildeten Hohlkörpers unter die Flüssigkeitsoberfläche hineinragt. In diesem Fall kann die Schutzhülse die Form eines Deckels für das Gefäß aufweisen, der manuell oder automatisch von oben auf das Gefäß aufgesetzt wird. In einem darauffolgenden Schritt wird die magnetische Vorrichtung in den von oben offenen Hohlkörper des Deckels eingeführt, so daß zumindest ein Teil der Vorrichtung unterhalb der Flüssigkeitsoberfläche zu liegen kommt.

Bei der Vorrichtung kann es sich prinzipiell um eine permanent magnetische oder eine magnetisierbare, zum Beispiel elektromagnetische Vorrichtung handeln. Sofern es sich bei der Vorrichtung um eine permanent magnetische Vorrichtung handelt, wandern die suspendierten Teilchen nach Eintauchen der Vorrichtung an die Grenzfläche zwischen der Flüssigkeit und der Schutzhülse und scheiden sich dort ab. Für den Fall, daß die Vorrichtung magnetisierbar ist, zum Beispiel wenn es sich um einen Elektromagneten handelt, findet diese Abscheidung nach Anlegen eines Magnetfeldes an die Vorrichtung statt.

Falls erforderlich oder gewünscht können die Teilchen, zum Beispiel durch Anlegen eines außerhalb des Gefäßes liegenden Magnetfeldes von der Schutzhülse entfernt und resuspendiert werden, gefolgt von einer erneuten Abscheidung an der Oberfläche der Schutzhülse. In einem weiteren Schritt des erfindungsgemäßen Verfahrens wird die Flüssigkeit, welche die nicht immobilisierten Komponenten enthält, von den an der Oberfläche der Schutzhülse haftenden Teilchen entfernt. Dies geschieht auf konventionelle Weise durch Absaugen der Flüssigkeit oder durch Ablassen der Flüssigkeit (zum Beispiel im Falle eines pipettenählichen Gefäßes).

In vielen Fällen wird den immobilisierten Teilchen noch ein Rest der Flüssigkeit mit den anderen Komponenten anhaften. Sofern dies bei der weiteren Bearbeitung stört, können die Teilchen durch Waschen von den anderen Komponenten befreit werden. Hierzu kann beispielsweise die Vorrichtung mit der Schutzhülse und den darauf befindlichen Teilchen in eine Waschflüssigkeit eingetaucht werden, die Immobilisierung der Teilchen durch Aufhebung der Magnetisierung oder Entfernung der magnetischen Vorrichtung aufgehoben oder ein außerhalb des Gefäßes befindlicher Magnet zur Anziehung der Teilchen verwendet werden. Durch erneutes Einführen der magnetischen Vorrichtung oder Anschalten des Magnetfeldes werden die Teilchen erneut an der Schutzhülse immobilisiert. Die Waschflüssigkeit kann - wie oben für die Probenflüssigkeit beschrieben - entfernt werden.

Das erfindungsgemäße Verfahren zeichnet sich dadurch aus, daß die Resuspension der magnetischen Teilchen besonders effektiv gestaltet werden kann. Es hat sich nämlich herausgestellt, daß bei langsamem Herausziehen der magnetischen Vorrichtung aus der Schutzhülse, solange diese noch in die Flüssigkeit eintaucht, eine sehr viel bessere Resuspension erreicht wird, als wenn die Vorrichtung einfach von einem außerhalb des Gefäßes liegenden Magnet entfernt wird. Die Bewegungsgeschwindigkeit der magnetischen Vorrichtung aus der Schutzhülse beträgt hierzu bevorzugt 0.1 mm/sec. bis 50 mm/sec., besonders bevorzugt 0.5 mm/sec bis 10 mm/sec. Diese Geschwindigkeiten können mittels einer Hubvorrichtung, die sich an einen XYZ-Arm eines automatischen Pipettierapparates befindet, auf einfache Weise realisiert werden, so daß keinerlei Zusatzmodule notwendig sind.

Die nun in gereinigter Form vorliegenden Komponenten können einer weiteren Verwendung zugeführt werden, zum Beispiel können sie nachgewiesen werden. Hierfür stehen für den Fachmann eine Reihe von Verfahren zur Verfügung. Die Komponenten können dazu von den magnetischen Teilchen deimmobilisiert (bei Nukleinsäuren beispielsweise durch Dehybridisierung) werden und von den Teilchen getrennt werden.

Gegenstand der Erfindung ist daher auch ein Verfahren zum Nachweis einer Komponente einer Flüssigkeit durch erfindungsgemäße Abtrennung dieser Komponente und anschließender Nachweis der Komponente.

Ebenfalls Gegenstand der Erfindung ist ein Gerät zur Abtrennung einer Komponente einer Flüssigkeit, die magnetische suspendierte Teilchen enthält, von anderen Komponenten enthaltend eine oder mehrere nach unten hinzu öffnende Gefäße zur Aufnahme der Flüssigkeit, eine oder mehrere Schutzhülsen aus nicht magnetischem Material, die in die Gefäße hineinragen können, eine magnetische Vorrichtung zum Einführen in die ein oder mehreren Schutzhülsen und eine Hubvorrichtung zum Einbringen der Schutzhülse und der magnetischen Vorrichtung in das Gefäß beziehungsweise Entfernen aus dem Gefäß, eine Vorrichtung zum Ablassen oder Absaugen von Flüssigkeit aus den ein oder mehreren Gefäßen. Die Gefäße und Schutzhülsen können in Magazinen auf Vorrat gehalten werden. Sobald eine Abtrennung erfolgen soll, wird dem Magazin eine Schutzhülse entnommen und zusammen mit oder getrennt von der magnetischen Vorrichtung durch die Hubvorrichtung in das Gefäß eingebracht. Die Hubvorrichtung kann beispielsweise an einem XYZ-Arm eines Pipettierroboters, zum Beispiel der Firma Tecan, angebracht sein.

Die magnetische Vorrichtung gemäß der Erfindung ist bevorzugt aus einer sandwichartigen Anordnung von kleinen Stabmagneten aufgebaut. Dabei sind die Pole jeweils alternierend in Hubrichtung angeordnet (Figur 1). Im Prinzip kann die Anordnung der einzelnen Stabmagnete wie in EP-A-0 136 126 geschehen, jedoch nicht außerhalb der Gefäße. In einer bevorzugten Ausführungsform (Figur 2) sind die Stabmagnete vertikal so angeordnet, daß die gleichen Pole aufeinander zuzeigen. Besonders bevorzugt sind die einzelnen Stabmagnete durch Stahlblättchen voneinander getrennt. In einer Ausführungsform mit Elektromagneten sind diese einzeln horizontal angeordnet, wobei die Pole in übereinanderliegenden Ebenen sternförmig gegeneinander versetzt sind. Dies gewährleistet eine gleichmäßige Anlagerung der Magnetpartikel. Für diesen Fall ist die Hubvorrichtung bevorzugt zusätzlich noch drehbar.

In dem erfindungsgemäßen Gerät kann zusätzlich zu der magnetischen Vorrichtung noch ein zweiter oder weiterer Magnet außerhalb des Gefäßes vorgesehen sein. Diese sind beispielsweise zum Resuspendieren der magnetischen Teilchen vorteilhaft verwendbar.

Eine besonders vorteilhafte Ausführungsform ergibt sich, wenn Form und Inhalt des Gefäßes und der Schutzhülse sowie das Flüssigkeitsvolumen optimal aufeinander abgestimmt sind. Besonders bevorzugt wird die Form der Schutzhülse so gewählt, daß ihre äußere Oberfläche immer den ungefähr gleichen Abstand zur inneren Oberfläche des Gefäßes aufweist. Eine beispielhafte Anordnung besteht zum Beispiel aus zwei ineinander gestellten zylindrischen Formen. Der von diesen Wänden gebildete Zwischenraum kann durch das Flüssigkeitsvolumen ausgefüllt werden. Dies bedeutet, daß vor Einbringen der Schutzhülse in das Gefäß nur soviel Flüssigkeit in das Gefäß eingefüllt werden darf, als dieses Volumen ausmacht. Beim Einführen der Schutzhülse in das Gefäß wird dann die Flüssigkeit zwischen die Zylinderwände gedrückt.

In dieser Ausführungsform, in der der zwischen der Schutzhülse und der inneren Oberfläche des Gefäßes gebildete Raum durch Einführen der Schutzhülse durch Probenflüssigkeit gefüllt wird, ist die Verwendung von elektromagnetischen Vorrichtungen besonders bevorzugt. Durch die Verteilung der Flüssigkeit in einer relativ dünnen Schicht um die magnetische Vorrichtung ist die mittels Elektromagneten erreichbare Feldstärke ausreichend. Dies war bei den im Stand der Technik beschriebenen Anordnungen nicht möglich.

Die Schutzhülse enthält außerdem bevorzugt Mittel, mit denen die Schutzhülse wieder aus dem Gefäß entfernt werden kann. Hierbei handelt es sich bevorzugt um Einbauten im inneren Teil der Schutzhülse, in den auch die Hubvorrichtung eingreift.

Das erfindungsgemäße Verfahren hat den Vorteil, daß es sehr leicht automatisiert werden kann. Außerdem ist bei der Erfindung vorteilhaft, daß die Resuspendierung besonders einfach und effektiv durchgeführt werden kann. Überraschenderweise werden die angezogenen Partikel durch Hub- oder Drehbewegung des Magneten besonders effektiv wieder resuspendiert. Dies wird insbesondere beim langsamen Auf-und Abbewegen der magnetischen Vorrichtung erreicht. Darüber hinaus wird durch die Verwendung einer Schutzhülse gewährleistet, daß die Kontaminationsgefahr, die durch Aerosole besteht und bei der Zellisolierung mit anschließender Amplifikation von Nukleinsäuren besonders schwerwiegend ist, weitgehend reduziert werden kann. Die Schutzhülse kann dafür als Wegwerfteil konzipiert und gegebenenfalls in doppelter Funktion auch als Deckel für das Gefäß verwendet werden. Durch einen Schichtaufbau aus kleinen Stabmagneten wird gewährleistet, daß die Magnetpartikel sich auf einer breiten Fläche niederschlagen, so daß das Waschen und Resuspendieren einfacher durchzuführen ist.

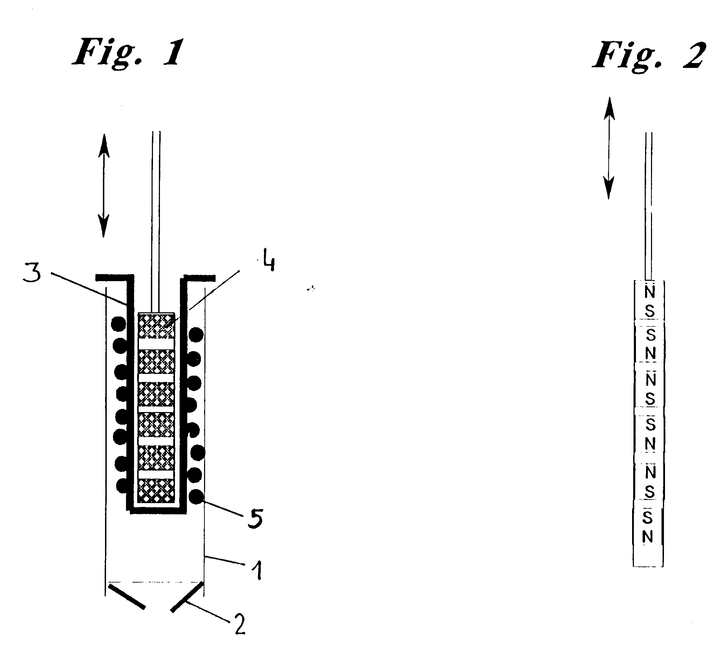

In Figur 1 ist ein Kunststoffgefäß (1) mit geöffneter Gummidichtlippe (2) gezeigt, in welchen eine Schutzhülse (3) hineinragt, in welcher sich wiederum eine aus - im Beispielsfall - sechs Stabmagneten zusammengesetzte magnetische Vorrichtung (4) befindet. Solche Kunststoffgefäße können mit Spritzgußverfahren hergestellt werden. Sie haben einen im wesentlichen zylindrischen Grundkörper und einen Boden, in den mindestens zwei Löcher eingelassen sind. Die Gummidichtlippe hat bevorzugt die Form einer Gummischeibe mit einer Öffnung, die sich nicht mit den Löchern des Bodens des Kunststoffgefäßes deckt. Duch Anlegen von Unterdruck am Gefäßboden wird die Gummidichtlippe vom Boden weggezogen und die Flüssigkeit kann durch die Löcher im Boden austreten. An der Außenwand der Schutzhülse (3) sind Magnetpartikel (5) in relativ gleichmäßiger Form verteilt.
In Figur 2 ist eine magnetische Vorrichtung gezeigt, bei der sechs Stabmagnete alternierend gepolt angeordnet sind.

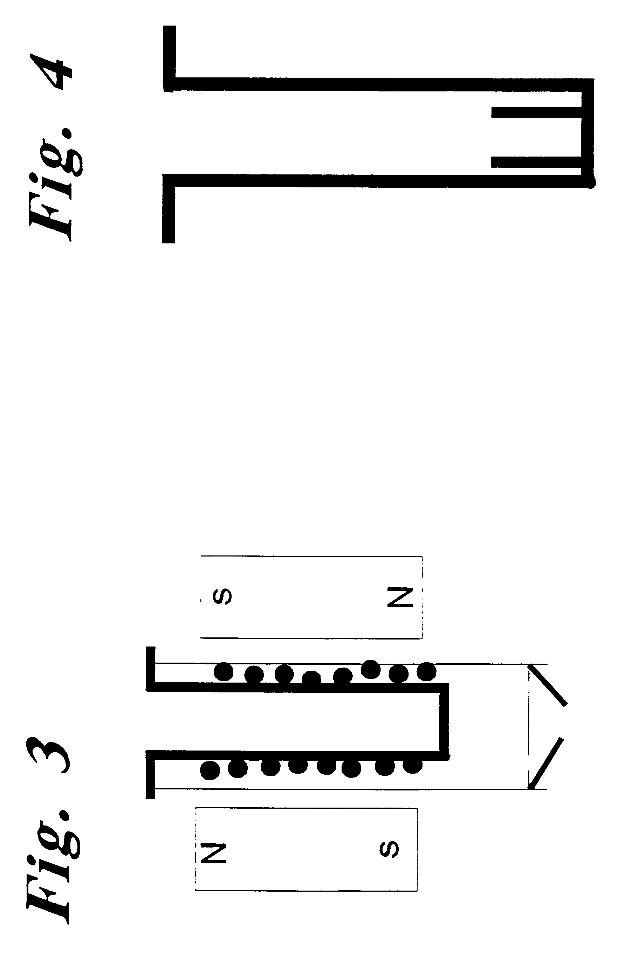

In Figur 3 ist ein Gefäß mit Schutzhülse und magnetischen Teilchen gezeigt, wobei zusätzlich außen an der Gefäßwand Magnete angebracht sind.
In Figur 4 ist eine Schutzhülse im Querschnitt gezeigt, bei der sich im Innenraum Mittel zur Entfernung der Schutzhülse aus dem Gefäß befinden.

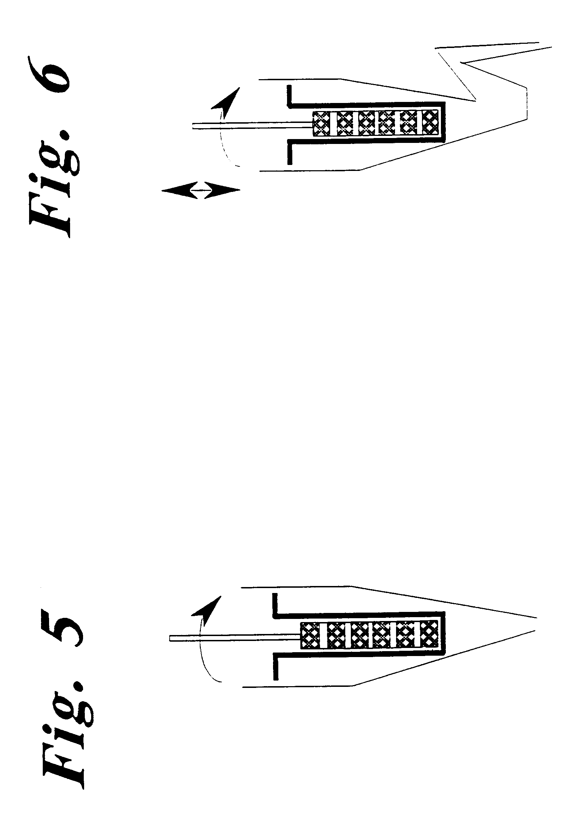

In Figur 5 ist eine erfindungsgemäße Vorrichtung gezeigt, bei der das Gefäß die Form einer Pipettenspitze hat.
In Figur 6 ist eine davon abgeleitete Ausführungsform gezeigt, bei der durch einen Syphon dem Verlust von magnetischen Teilchen bei einem Transfer begegnet wird.

## Patentansprüche

1. Verfahren zur Abtrennung einer Komponente einer Flüssigkeit von anderen Komponenten durch
a) Immobilisierung der Komponente an magnetischen suspendierten Teilchen in einem nach unten hin zu öffnenden Gefäß;
b) Eintauchen einer magnetischen Vorrichtung in das Gefäß, wobei die Vorrichtung gegenüber der Flüssigkeit durch eine Schutzhülse aus nicht magnetischem Material abgetrennt ist, und
c) Entfernen nicht immobilisierter Komponenten durch Absaugen oder Ablassen der Flüssigkeit aus dem Gefäß.

2. Verfahren gemäß Anspruch 1, dadurch gekennzeichnet, daß die magnetische Vorrichtung sandwichartig aus mehreren alternierend gepolten Permanentmagneten oder Elektromagneten aufgebaut ist.

3. Verfahren gemäß einem der Ansprüche 1 und 2, dadurch gekennzeichnet, daß die Komponente aus Zellen besteht.

4. Verfahren gemäß Anspruch 1, dadurch gekennzeichnet, daß die Komponente aus Nukleinsäuren besteht.

5. Verfahren gemäß Anspruch 1, dadurch gekennzeichnet, daß anschließend an Schritt c) die Teilchen durch Drehen oder/und Heben der magnetischen Vorrichtung resuspendiert werden.

6. Verfahren zum Nachweis einer Komponente einer Flüssigkeit, dadurch gekennzeichnet, daß die Komponente nach einem Verfahren gemäß Ansprüche 1-5 von anderen Komponenten abgetrennt wird und anschließend nachgewiesen wird.

7. Gerät zur Abtrennung einer Komponente einer Flüssigkeit, die magnetische suspendierte Teilchen enthält, von anderen Komponenten enthaltend
- ein oder mehrere nach unten hin zu öffnende Gefäße zur Aufnahme der Flüssigkeit,
- eine oder mehrere Schutzhülsen aus nicht magnetischem Material, die in die Gefäße hineinragen können,
- eine magnetische Vorrichtung, zum Einführen in die ein oder mehreren Schutzhülsen und
- eine Hubvorrichtung zum Einbringen der Schutzhülse und der magnetischen Vorrichtung in das Gefäß bzw. zum Entfernen aus dem Gefäß,
- eine Vorrichtung zum Ablassen oder Absaugen von Flüssigkeit aus den ein oder mehreren Gefäßen.

8. Gerät gemäß Anspruch 7, dadurch gekennzeichnet, daß die magnetische Vorrichtung aus einem Sandwich kleiner Stabmagneten oder Elektromagneten aufgebaut ist.

9. Gerät gemaß Anspruch 7, dadurch gekennzeichnet, daß die Schutzhülse die Form eines Deckels für das Gefäß hat.

10. Gerät gemäß Anspruch 7, dadurch gekennzeichnet, daß es zum Resuspendieren zusätzlich einen Magneten außerhalb des Gefäßes besitzt.

## Claims

1. Method for separating a component of a liquid from other components by
a) immobilizing the component on suspended magnetic particles in a vessel which can be opened downwards,
b) immersing a magnetic device in the vessel, the device being separated from the liquid by means of a protective sleeve made of a non-magnetic material and
c) removing non-immobilized components by aspirating or draining the liquid from the vessel.

2. Method as claimed in claim 1, wherein the magnetic device is composed of a sandwich of several permanent magnets or electromagnets with alternating poles.

3. Method as claimed in one of the claims 1 and 2, wherein the components are cells.

4. Method as claimed in claim 1, wherein the components are nucleic acids.

5. Method as claimed in claim 1, wherein subsequently to step c) the particles are resuspended by rotating and/or lifting the magnetic device.

6. Method for detecting a component of a liquid, wherein the component is separated from other components by a method as claimed in claims 1 - 5 and subsequently detected.

7. Device for separating a component of a liquid which contains suspended magnetic particles from other components comprising
- one or several vessels for receiving the liquid which can be opened downwards,
- one or several protective sleeves made of non-magnetic material which can extend into the vessels,
- a magnetic device which can be inserted into the one or several protective sleeves and
- a lifting device for introducing and removing the protective sleeve and the magnetic device into and from the vessel
- a device for draining or aspirating liquid from the one or several vessels.

8. Device as claimed in claim 7, wherein the magnetic device is comprised of a sandwich of small bar magnets or electromagnets.

9. Device as claimed in claim 7, wherein the protective sleeve has the shape of a lid for the vessel.

10. Device as claimed in claim 7, wherein it has an additional magnet outside the vessel for resuspension.

## Revendications

1. Procédé de séparation d'un composant d'un liquide d'autres composants par:
a) immobilisation du composant sur des particules en suspension magnétique dans un récipient pouvant être ouvert vers le bas;
b) immersion d'un dispositif magnétique dans le récipient, le dispositif étant séparé du liquide par une gaine de protection en matériau non magnétique, et
c) élimination des composants non immobilisés par aspiration ou purge du liquide hors du récipient.

2. Procédé selon la revendication 1, caractérisé en ce que le dispositif magnétique est constitué de plusieurs amants permanents ou électro-aimants à pôles alternés disposés en sandwich.

3. Procédé selon l'une des revendications 1 et 2, caractérisé en ce que le composant est constitué de cellules.

4. Procédé selon la revendication 1, caractérisé en ce que le composant est constitué d'acides nucléiques.

5. Procédé selon la revendication 1, caractérisé en ce que, à la suite de l'étape c), les particules sont remises en suspension par rotation ou/et soulèvement du dispositif magnétique.

6. Procédé pour la détermination d'un composant d'un liquide, caractérisé en ce que le composant est séparé des autres composants selon un procédé selon les revendications 1 à 5, et est déterminé ensuite.

7. Appareil pour la séparation d'un composant d'un liquide contenant des particules en suspension magnétique des autres composants comportant
- un ou plusieurs récipients pouvant être ouverts vers le bas pour recevoir le liquide,
- une ou plusieurs gaines de protection en matériau non magnétique, qui peuvent être insérées dans le récipient,
- un dispositif magnétique pour son introduction dans la ou les gaines de protection,
- un dispositif de levage pour placer la gaine de protection du dispositif magnétique dans le récipient resp. pour l'enlever du récipient,
- un dispositif pour purger ou aspirer le liquide du récipient ou des différents récipients.

8. Appareil selon la revendication 7, caractérisé en ce que le dispositif magnétique est formé d'un empilement de petits barreaux magnétiques ou d'électro-aimants.

9. Appareil selon la revendication 7, caractérisé en ce que la gaine de protection a la forme d'un couvercle pour le récipient.

10. Appareil selon la revendication 7, caractérisé en ce qu'il comporte en outre un aimant en dehors du récipient pour la remise en suspension.
